# FASCICULE DE BREVET EUROPEEN

(11) **EP 3 528 874 B1**
(45) Date de publication et mention de la délivrance du brevet: **07.12.2022**
(21) Numéro de dépôt: 17794679.5
(22) Date de dépôt: 18.10.2017
(51) Int. Cl.: A61M 16/00, F04D 25/08, F04D 29/58

(54) **APPAREIL D'ASSISTANCE RESPIRATOIRE A SYSTÈME DE REFROIDISSEMENT INTÉGRÉ**
ATEMHILFEVORRICHTUNG MIT INTEGRIERTEM KÜHLSYSTEM
RESPIRATORY ASSISTANCE DEVICE WITH INTEGRATED COOLING SYSTEM

(30) Priorité: 19.10.2016 FR 1660113
(43) Date de publication de la demande: 28.08.2019
(73) Titulaire: Airfan, 31770 Colomiers (FR)
(72) Inventeur: PRAT, Xavier, 31270 Villeneuve Tolosane (FR)
(74) Mandataire: Junca, Eric
(86) Numéro de dépôt international: PCT/EP2017/076532
(87) Numéro de publication internationale: WO 2018/073275

(56) Documents cités:
- FR-A1- 2 973 846
- US-A1- 2008 304 986
- US-A1- 2014 069 432

## Description

### DOMAINE DE L'INVENTION ET ÉTAT DE LA TECHNIQUE

La présente invention se rapporte à un appareil de production régulée de gaz destiné principalement à l'assistance respiratoire d'un patient et intégrant un système de refroidissement d'un moteur électrique utilisé dans cet appareil. Le gaz est constitué par l'air pulsé délivré en sortie de l'appareil.

Les appareils dédiés à l'assistance respiratoire forment une enceinte qui loge notamment un ventilateur centrifuge composé essentiellement d'un moteur électrique couplé à une roue à ailettes, cette roue entraînée par le moteur fournissant le gaz pulsé dans des conditions de pression et de débit régulées. L'enceinte de l'appareil boîtier présente une entrée d'air qui alimente la roue à ailettes pour fournir le gaz pulsé à travers un orifice de sortie. De tels appareils sont décrits par exemple dans les documents de brevet FR 2 908 482, FR 2 953 142 ou FR 2 973 846 au nom du présent déposant.

Les moteurs électriques de ventilateur de ce type d'appareil présentent un très haut rendement (typiquement de l'ordre de 90%) avec une faible puissance (typiquement inférieure à 80 watts). Ils fonctionnent à vitesse de rotation élevée, (typiquement jusqu'à 80 000 tours/min) ce qui provoque un échauffement important de leur environnement, en particulier dans les paliers de guidage du moteur, notamment à faible débit du ventilateur.

Dans les solutions actuellement mises en œuvre, le refroidissement des moteurs électriques se fait usuellement extérieurement par une circulation d'air le long du carter du moteur avant d'intégrer le circuit d'air du ventilateur dédié au patient.

Afin d'améliorer le refroidissement du moteur, le brevet FR 2 973 846 propose en particulier la formation de trous dans les parois transversales du carter moteur afin de réaliser également un écoulement de gaz de refroidissement provenant du ventilateur et circulant dans le carter moteur.

Cependant, le refroidissement externe le long du carter moteur nécessite la mise en place d'un boîtier enveloppant le ventilateur et le moteur ce qui rend complexe la réalisation de l'appareil.

De plus, la circulation d'air destinée à l'évacuation thermique de la chaleur au cœur des roulements des paliers pollue la graisse et la châsse, ce qui détruit la qualité de lubrification des roulements et réduit en conséquence la durée de vie de ces roulements et donc du moteur. Accessoirement, l'absence de graisse génère une élévation de température dans les roulements ce qui, à la longue, peut endommager le moteur jusqu'à la casse.

### EXPOSÉ DE L'INVENTION

L'invention vise à remédier à ces inconvénients, à savoir permettre l'évacuation de la chaleur générée par le moteur pour abaisser la température de fonctionnement nominal de l'appareil tout en assurant un bon fonctionnement du moteur et une fiabilité augmentée de l'appareil, en particulier sans pic de température, et donc une durée de vie maximisée du moteur. Pour ce faire, l'air destiné à refroidir le moteur est prélevé du flux entraîné par la roue à ailettes provenant directement du flux d'air pénétrant dans l'enceinte et les roulements du moteur sont protégés des courants et de la circulation d'air par étanchéification entre la partie tournante et la partie fixe du moteur.

A ce titre, la présente invention a pour objet un appareil d'assistance respiratoire à système de refroidissement intégré. Un tel appareil comporte une enceinte logeant un ventilateur à moteur électrique couplé à une roue à ailettes et présentant une entrée et au moins deux sorties d'air, une sortie de gaz patient et une sortie d'air de refroidissement du moteur électrique d'axe longitudinal délimité par un carter à parois tubulaires longitudinales et flasques d'extrémité transversaux. Ledit moteur comporte un stator monté sur la paroi tubulaire du carter et un rotor muni d'un arbre moteur longitudinal traversant au moins un des flasques transversaux du carter via des paliers de guidage pour entraîner en rotation la roue à ailettes logée dans une volute de réception d'air.

Dans cet appareil, un flux d'air provenant directement de l'entrée de l'enceinte est apte à être brassé par les ailettes de la roue, puis pulsée vers la sortie de gaz patient via un conduit de sortie couplé à la volute. Une fraction de cet air est prélevée dans la volute et s'écoule entre le rotor et le stator du moteur à travers le système de refroidissement comportant des orifices formés dans chacun des flasques transversaux et un canal d'évacuation d'air couplé d'une part à la paroi tubulaire du carter et d'autre part à la sortie d'air de refroidissement de l'enceinte.

De plus, dans ce système, des rondelles d'étanchéité, solidarisées en recouvrement des flasques transversaux, sont centrées sur l'arbre moteur et sont dimensionnées de sorte que ces rondelles protègent les paliers de l'air circulant et sortant du moteur en s'étendant jusqu'au plus près de l'arbre moteur sans générer de frottement ni obturer les orifices du système de refroidissement afin de ne pas perturber la circulation d'air.

Selon des modes de réalisation avantageux:
- l'arbre moteur traversant de part en part le flasque d'extrémité du carter moteur couplé à la volute dit flasque avant, et pénétrant l'autre flasque d'extrémité dit flasque arrière jusqu'au droit ou en deçà de la face arrière du flasque arrière, la rondelle d'étanchéité solidarisée au flasque avant est percée en son centre selon une découpe circulaire ajustée sur l'arbre moteur et la rondelle d'étanchéité solidarisée au flasque arrière forme un capuchon non percé;
- l'arbre moteur traversant de part en part les deux flasques d'extrémité du carter moteur, les rondelles d'étanchéité sont percées en leur centre selon une découpe circulaire ajustée sur l'arbre moteur avec un jeu sensiblement nul;
- les rondelles sont solidarisées aux flasques d'extrémité du carter moteur par une technologie de solidarisation choisie entre collage, fusion thermique, fusion par ultrasons et soudage laser;
- la ou les rondelles présentent un serrage adapté sur l'arbre moteur après solidarisation et rodage sur l'arbre moteur, une étanchéité optimisée des paliers étant ainsi réalisée sans générer de frottements néfastes;
- les orifices du système de refroidissement sont régulièrement et circulairement répartis dans chacun des flasques d'extrémité du carter moteur en se positionnant de manière longitudinalement alignée;
- les orifices du système de refroidissement présentent un diamètre adapté pour autoriser un débit d'air de refroidissement du moteur suffisant sans diminuer sensiblement le débit de gaz destiné au patient;
- le moteur présente une perméabilité à l'air comprise entre 5 et 50 l/min sous une pression de 40 à 100 mbar ajustée par le diamètre et le nombre d'orifices formés dans les flasques d'extrémité du carter moteur.

### PRÉSENTATION DES FIGURES

D'autres avantages et caractéristiques de l'invention pourront apparaître lors de la description détaillée qui suit, en référence aux figures annexées qui représentent, respectivement :
- la figure 1, une vue en coupe d'un exemple de ventilateur pour un appareil d'assistance respiratoire selon l'invention ;
- la figure 2, une vue en coupe selon le plan AA du flasque d'extrémité avant du carter moteur du ventilateur selon la figure 1; et
- la figure 3, une vue en coupe schématique d'un exemple d'enceinte d'appareil d'assistance respiratoire logeant le ventilateur selon la figure 1.

**Sur** les figures, des signes de référence identiques se rapportent à un même élément et renvoient aux passages de la description détaillée qui le spécifient.

### DESCRIPTION DETAILLEE

Les termes « externe » et « interne» qualifient, dans le présent texte, les faces d'un même élément tournées respectivement vers l'environnement externe du moteur et vers l'espace interne de ce moteur. Par ailleurs, le qualificatif « axial » ou « longitudinal » se rapporte aux éléments s'étendant principalement selon l'arbre longitudinal du moteur et « transversal » aux éléments s'étendant perpendiculairement à cet arbre. De plus, les qualificatifs « avant » et « arrière » se rapportent à l'ordre dans lesquels des éléments semblables, par exemple les flasques du carter moteur, sont atteints par la circulation d'air. De même, le qualificatif « au-dessus » localise un élément ou un espace atteint par la circulation d'air avant un autre élément.

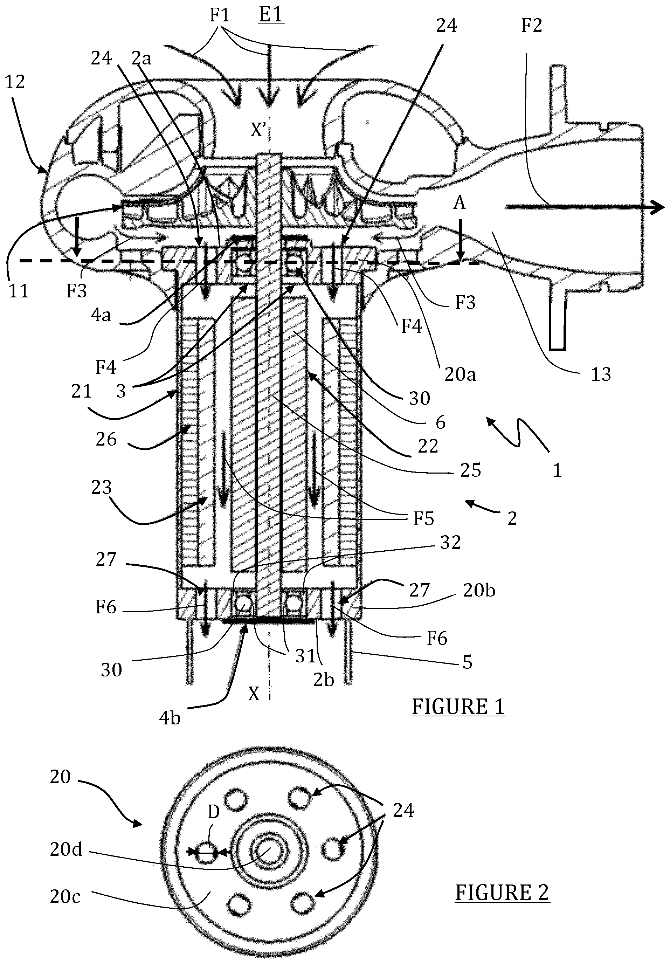

La vue en coupe du ventilateur 1 de la figure 1 montre un ensemble de mise en circulation de l'air constitué d'une roue à ailettes 11 logée dans une volute 12 et entraîné par un moteur 2 protégé dans un carter 20. Il apparaît que les flux d'air (flèches F1) qui pénètrent dans la roue à ailettes 11 viennent directement de l'espace E1 situé au-dessus de la volute 12 et de la roue à ailettes 11. Ces flux d'air F1 sont entraînés par la roue 11 qui génère une surpression pour transmettre un flux de gaz (flèche F2) dans un conduit de sortie en expansion 13 destiné au patient.

Une fraction des flux d'air en surpression est prélevée dans la volute 12, au dos de la roue à ailettes 11, pour être dirigée (flèches F3) vers le flasque d'extrémité avant 20a du carter moteur 20. Plus précisément, ce carter moteur 20 est constitué de flasques d'extrémité avant 20a et arrière 20b ainsi que d'une paroi tubulaire 21 formée entre les flasques 20a et 20b, les flasques 20a et 20b s'étendant de manière transversale par rapport à la paroi tubulaire 21 qui s'étend longitudinalement.

Le carter moteur 20 loge le moteur 2 qui comporte un rotor 22 intégrant un aimant 6 fixé sur un arbre moteur d'entraînement longitudinal 25 d'axe X'X couplé à la roue 11 pour l'entraîner en rotation, et un stator 23 s'étendant de manière interne le long de la paroi tubulaire 21 via des tôles support 26.

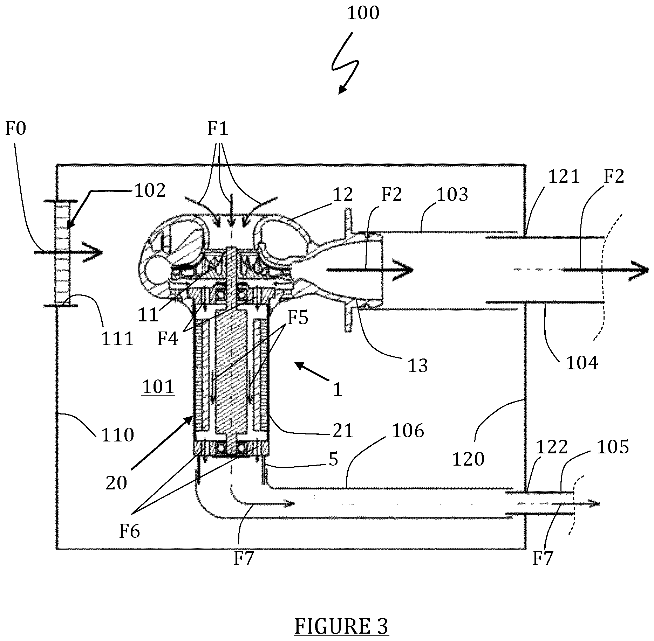

Dans ces conditions, la fraction d'air prélevée dans la volute 12 (flèches F3) traverse (flèches F4) le flasque avant 20a par des orifices 24 régulièrement répartis dans le flasque avant 20a (cf. figure 2). Puis cet air circule dans le moteur 2 entre le rotor 22 et le stator 23 (flèches F5), avant de ressortir du moteur 2 par des orifices 27 formés dans le flasque arrière 20b (flèches F6). Une paroi cylindrique 5 servant à canaliser le flux d'air de refroidissement prolonge la paroi tubulaire 21 et est couplée à un canal d'évacuation (cf. le canal d'évacuation 106 de la figure 3). Les orifices 24 et 27 longitudinalement alignés et cette paroi cylindrique 5 constituent le système de refroidissement interne du moteur 2.

Dans cet exemple, l'arbre moteur 25 traverse le flasque avant 20a et vient en retrait de la face externe 2b du flasque d'extrémité arrière 20b respectivement par le jeu de paliers 3, ici à roulement à billes 30. Chaque palier 3 est constitué d'une bague tournante 31 et d'une bague fixe 32, respectivement solidaire de l'arbre moteur d'entraînement 25 et du flasque d'extrémité avant 20a et arrière 20b.

Selon l'invention, les faces externes 2a et 2b, respectivement des flasques d'extrémité 20a et 20b, sont recouverts centralement de rondelles d'étanchéité 4a et 4b solidarisées aux faces externes 2a et 2b. D'autres moyens d'étanchéité, comme des roulements flasqués ou des joints spi, internes aux paliers, entraîneraient des efforts de frottement rédhibitoires au regard de la faible puissance développée par les moteurs utilisés dans ce type d'application et du rendement global élevé pour ce type d'application.

Ces rondelles d'étanchéité 4a et 4b, formées respectivement par une couronne et un disque en matériau plastique, sont donc centrées sur l'axe X'X et ont un diamètre suffisant pour recouvrir les paliers 3. Les rondelles 4a et 4b sont solidarisées par collage dans cet exemple, mais d'autres technologies de liaison (par exemple par fusion thermique, par ultrasons ou par soudage laser) sont alternativement utilisables.

Dans l'exemple, l'arbre moteur 25 traversant totalement le flasque d'extrémité avant 20a, la rondelle 4a est percée en son centre de sorte qu'elle soit ajustée sur l'arbre moteur 25 avec un serrage préalablement rodé sur l'arbre moteur 25 dans des conditions de durée et de température optimisées par étalonnage.

S'agissant du flasque d'extrémité arrière 20b, l'arbre moteur 25 ne traversant pas totalement ce flasque 20b, la rondelle 4b n'est pas percée et forme alors un capuchon hermétique.

Dans l'exemple de réalisation, les rondelles 4a et 4b s'étendent sur les faces externes 2a et 2b tout en restant à distance des orifices 24 qui traversent les flasques 20a et 20b.

En référence à la vue en coupe de la figure 2, illustrant le flasque d'extrémité avant 20a, il apparait que les orifices 24 sont régulièrement et circulairement répartis dans ce flasque 20a (et donc également dans le flasque d'extrémité arrière 20b de la figure 1).

Les orifices 24, au nombre de six dans l'exemple, ont un diamètre « D » adapté afin de permettre la mise en place d'un débit d'air de refroidissement du moteur 2 (flèches F4, F5, F6) suffisant sans diminuer sensiblement le débit de gaz destiné au patient (flèche F2).

Dans ces conditions, le moteur 2 présente une perméabilité à l'air comprise entre 5 et 50 l/min sous une pression de 85 mbar et plus précisément déterminée par la valeur du diamètre « D» et le nombre d'orifices 24 formés dans les flasques d'extrémité 20a et 20b. Dans l'exemple de réalisation illustré, une perméabilité à l'air de 30 l/min est obtenue avec un diamètre de 2,5 mm pour les six orifices 24. Ainsi, en fonction des domaines d'utilisation, le flux d'air de refroidissement est adaptable grâce au nombre et à la dimension des orifices 24 dans les flasques d'extrémité avant 20a et arrière 20b.

Le flasque d'extrémité avant 20a (comme le flasque d'extrémité arrière 20b) présente un alésage central selon la découpe circulaire 20d afin de laisser passer l'arbre moteur 2.

En référence à la vue en coupe schématique de la figure 3 d'un exemple d'enceinte 100 d'appareil d'assistance respiratoire selon l'invention, le ventilateur 1 de la figure 1 est alors illustré tel que logé dans cette enceinte 100.

Le ventilateur 1 est monté directement dans l'enceinte 100 de l'appareil d'assistance respiratoire à l'aide de moyens de fixation par clipsage, vissage ou équivalent, des attaches souples amovibles, etc. Une telle architecture permet d'optimiser l'encombrement et la masse de l'appareil d'assistance respiratoire.

L'air provenant de l'extérieur de l'enceinte 100 (flèche F0) traverse un filtre 102 absorbant les particules polluantes et monté dans un orifice d'entrée 111 d'une paroi d'entrée 110. Cet air F0 est aspiré dans la volute 12 (flèches F1) par la rotation de la roue à ailettes 11. Comme présenté plus haut en référence à la figure 1, l'air aspiré ressort sous forme de gaz pulsé (flèches F2) dans le conduit de sortie en expansion 13 couplé, via un conduit intermédiaire avant 103, à une tubulure de sortie 104 destinée au patient. Cette tubulure 104 traverse la paroi de sortie 120 de l'enceinte 100 opposée à la paroi d'entrée 110 par un orifice de sortie avant 121.

Par ailleurs, l'air de refroidissement moteur (flèches F4, F5, F6) est dirigé par le système de refroidissement intégré depuis la paroi cylindrique 5 prolongeant la paroi tubulaire 21 (flèches F7), vers le canal d'évacuation 105 - qui traverse la paroi de sortie 120 par un orifice de sortie arrière 122 - via un conduit intermédiaire arrière 106. Dans l'exemple, les conduits intermédiaires 103 et 106 ainsi que la tubulure de sortie 104 et le canal d'évacuation 105 sont parallèles afin de rationnaliser l'architecture vers la paroi de sortie 120.

Le flux d'air de refroidissement est ainsi canalisé par le conduit arrière 106, ce qui permet également de diminuer les calories diffusées à l'intérieur du système d'assistance respiratoire en mettant en œuvre une liaison du conduit arrière 106 directement vers l'extérieur.

L'invention n'est pas limitée aux exemples de réalisation décrits et représentés. En effet, d'autres configurations pour les sorties d'air et de gaz sont possibles en respectant l'indépendance du gaz de sortie patient par rapport à l'air de refroidissement moteur. Le gaz destiné au patient est ainsi maintenu à température ambiante, indépendamment de la température du moteur.

Par ailleurs, l'air de refroidissement peut également être évacué du moteur par une fente prévue en général pour le passage des fils électriques d'alimentation moteur. Dans ce cas, l'air provenant de cette fente est également pris en charge par la paroi cylindrique vers le canal d'évacuation.

## Revendications

1. Appareil d'assistance respiratoire à système de refroidissement intégré comportant une enceinte (100) logeant un ventilateur (1) à moteur électrique (2) couplé à une roue à ailettes (11) et présentant une entrée (111) et au moins deux sorties d'air (121, 122), une sortie de gaz patient (121) et une sortie d'air de refroidissement (122) du moteur électrique (2) d'axe longitudinal (X'X) et délimité par un carter (20) à paroi tubulaire longitudinale (21) et flasques d'extrémité transversaux (20a, 20b), ledit moteur (2) comportant un stator (23) monté sur la paroi tubulaire (21) du carter (20) et un rotor (22) monté sur un arbre moteur longitudinal (25) traversant au moins l'un des flasques transversaux (20a, 20b) du carter (20) via des paliers de guidage (3) pour entraîner en rotation la roue à ailettes (11) logée dans une volute de réception d'air (12), et dans lequel de l'air brassé par les ailettes de roue (11) est pulsé vers la sortie de gaz patient (121) via un conduit de sortie (13) couplé à la volute (12), une fraction de cet air étant prélevée dans la volute (12) et s'écoule entre le rotor (22) et le stator (23) du moteur (2) à travers le système de refroidissement comportant des orifices (24, 27) formés dans chacun des flasques transversaux (20a, 20b), et un canal d'évacuation d'air (105) est couplé d'une part à la paroi tubulaire (21) du carter (20) et d'autre part à la sortie d'air de refroidissement (122) de l'enceinte (100), **caractérisé en ce que**, dans cet appareil, un flux d'air (F0) provenant directement de l'entrée (111) de l'enceinte (100) est aspiré (flèches F1) dans la volute (12) par la rotation de la roue à ailettes (11), ces flux d'air (flèches F1) venant directement de l'espace (E1) situé au-dessus de la volute (12) , et **en ce que** dans ce système des rondelles d'étanchéité (4a, 4b), solidarisées en recouvrement des flasques transversaux (20a, 20b), sont centrées sur l'arbre moteur (25) et sont dimensionnées de sorte que ces rondelles (4a, 4b) protègent les paliers (3) de l'air (F3, F4, F5) circulant et sortant du moteur (2) en s'étendant jusqu'au plus près de l'arbre moteur sans générer de frottement ni obturer les orifices (24, 27) du système de refroidissement.

2. Appareil d'assistance respiratoire selon la revendication 1, dans lequel l'arbre moteur (25) traversant de part en part le flasque d'extrémité du carter moteur (20) couplé à la volute (12) dit flasque avant (20a), et pénétrant l'autre flasque d'extrémité dit flasque arrière (20b) jusqu'au droit ou en deçà de la face arrière (2b) du flasque arrière (20b), la rondelle d'étanchéité (4a) solidarisée au flasque avant (20a) est percée en son centre selon une découpe circulaire ajustée sur l'arbre moteur (25) et la rondelle d'étanchéité (4b) solidarisée au flasque arrière (20b) forme un capuchon non percé.

3. Appareil d'assistance respiratoire selon la revendication 1, dans lequel l'arbre moteur (25) traversant de part en part les deux flasques d'extrémité (20a, 20b) du carter moteur (20), les rondelles d'étanchéité (4a, 4b) sont percées en leur centre selon une découpe circulaire ajustée sur l'arbre moteur (25) avec un jeu sensiblement nul.

4. Appareil d'assistance respiratoire selon l'une quelconque des revendications précédentes, dans lequel les rondelles (4a, 4b) sont solidarisées aux flasques d'extrémité du carter moteur (20) par une technologie de solidarisation choisie entre collage, fusion thermique, fusion par ultrasons et soudage laser

5. Appareil d'assistance respiratoire selon l'une quelconque des revendications précédentes, dans lequel la ou les rondelles (4a, 4b) présentent un serrage adapté sur l'arbre moteur (25) après solidarisation et rodage sur l'arbre moteur (25).

6. Appareil d'assistance respiratoire selon l'une quelconque des revendications précédentes, dans lequel les orifices (24, 27) du système de refroidissement sont régulièrement et circulairement répartis dans chacun des flasques d'extrémité (20a, 20b) du carter moteur (20) en se positionnant de manière longitudinalement alignée.

7. Appareil d'assistance respiratoire selon l'une quelconque des revendications précédentes, dans lequel les orifices (24, 27) du système de refroidissement présentent un diamètre (D) adapté pour autoriser un débit d'air de refroidissement du moteur (2) suffisant sans diminuer sensiblement le débit de gaz destiné au patient.

8. Appareil d'assistance respiratoire selon l'une quelconque des revendications précédentes, dans lequel le moteur électrique (2) présente une perméabilité à l'air comprise entre 5 et 50 l/min sous une pression de 40 à 100 mbar ajustée par le diamètre (D) et le nombre d'orifices formés dans les flasques d'extrémité (20a, 20b) du carter moteur (20).

## Patentansprüche

1. Atemunterstützungsgerät mit integriertem Kühlsystem, das einen Behälter (100) umfasst, der einen Ventilator (1) mit Elektromotor (2) aufnimmt, der mit einem Flügelrad (11) gekoppelt ist und einen Einlass (111) und mindestens zwei Luftauslässe (121, 122), einen Patientengasauslass (121) und einen Kühlluftauslass (122) des Elektromotors (2) mit einer Längsachse (X'X), aufweist und durch ein Gehäuse (20) mit einer längs verlaufenden rohrförmigen Wand (21) und quer verlaufenden Endflanschen (20a, 20b) begrenzt wird, wobei der Motor (2) einen Stator (23) aufweist, der an der rohrförmigen Wand (21) des Gehäuses (20) montiert ist, und einen Rotor (22), der an einer längs verlaufenden Motorwelle (25) montiert ist, die durch zumindest einen der quer verlaufenden Flansche (20a, 20b) des Gehäuses (20) über Führungslager (3) verläuft, um das in einer Luftaufnahmespirale (12) untergebrachte Flügelrad (11) zu drehen, und wobei die von den Radflügeln (11) umgewälzte Luft über eine mit der Spirale (12) gekoppelte Ausgangsleitung (13) zum Patientengasauslass (121) gepulst wird, wobei ein Teil dieser Luft aus der Spirale (12) entnommen wird und zwischen dem Rotor (22) und dem Stator (23) des Motors (2) durch das Kühlsystem mit Öffnungen (24, 27), die in jedem der quer verlaufenden Flansche (20a, 20b) ausgebildet sind, strömt, und wobei ein Luftabführkanal (105) einerseits mit der rohrförmigen Wand (21) des Gehäuses (20) und andererseits mit dem Kühlluftauslass (122) des Behälters (100) gekoppelt ist, **dadurch gekennzeichnet, dass** in diesem Gerät ein direkt vom Einlass (111) des Behälters (100) kommender Luftstrom (F0) in der Spirale (12) durch die Drehung des Flügelrads (11) angesaugt wird (Pfeile F1), wobei diese Luftströme (Pfeile F1) direkt aus dem Raum (E1) kommen, der sich über der Spirale (12) befindet, und dadurch, dass in diesem System Dichtungsscheiben (4a, 4b), die mit den quer verlaufenden Flanschen (20a, 20b) überlappend fest verbunden sind, auf der Motorwelle (25) zentriert und so dimensioniert sind, dass diese Scheiben (4a, 4b) die Lager (3) vor der Luft (F3, F4, F5), die im Motor (2) zirkuliert und aus diesem austritt, schützen, indem sie sich so nah wie möglich an die Motorwelle erstrecken, ohne Reibung zu erzeugen und ohne die Öffnungen (24, 27) des Kühlsystems zu verschließen.

2. Atemunterstützungsgerät nach Anspruch 1, wobei die Motorwelle (25) vollständig durch den mit der Spirale (12) gekoppelten Endflansch des Motorgehäuses (20), den sogenannten vorderen Flansch (20a), verläuft und in den anderen Endflansch, den sogenannten hinteren Flansch (20b), bis höchstens an die Rückseite (2b) des hinteren Flanschs (20b) eindringt, wobei die mit dem vorderen Flansch (20a) fest verbundene Dichtungsscheibe (4a) in ihrer Mitte gemäß einem kreisförmigen, an die Motorwelle (25) angepassten Ausschnitt durchbohrt ist und die mit dem hinteren Flansch (20b) fest verbundene Dichtungsscheibe (4b) eine nicht durchbohrte Kappe bildet.

3. Atemunterstützungsgerät nach Anspruch 1, wobei die Motorwelle (25) die beiden Endflansche (20a, 20b) des Motorgehäuses (20) vollständig durchdringt, wobei die Dichtungsscheiben (4a, 4b) in ihrer Mitte gemäß einem kreisförmigen, an die Motorwelle (25) mit einem Spiel von im Wesentlichen Null angepassten Ausschnitt durchbohrt sind.

4. Atemunterstützungsgerät nach einem der vorangehenden Ansprüche, wobei die Scheiben (4a, 4b) durch eine Verbindungstechnologie, die aus Kleben, thermischem Schweißen, Ultraschallschmelzen und Laserschweißen ausgewählt ist, fest mit den Endflanschen des Motorgehäuses (20) verbunden sind.

5. Atemunterstützungsgerät nach einem der vorangehenden Ansprüche, wobei die Scheibe(n) (4a, 4b) nach der Verbindung und dem Einlaufen auf der Motorwelle (25) eine passende Klemmung auf der Motorwelle (25) aufweist/aufweisen.

6. Atemunterstützungsgerät nach einem der vorangehenden Ansprüche, wobei die Öffnungen (24, 27) des Kühlsystems gleichmäßig und kreisförmig in jedem der Endflansche (20a, 20b) des Motorgehäuses (20) verteilt sind, indem sie in Längsrichtung ausgerichtet positioniert sind.

7. Atemunterstützungsgerät nach einem der vorangehenden Ansprüche, wobei die Öffnungen (24, 27) des Kühlsystems einen Durchmesser (D) aufweisen, der dazu geeignet ist, einen ausreichenden Kühlluftdurchsatz des Motors (2) zu ermöglichen, ohne den für den Patienten bestimmten Gasdurchsatz wesentlich zu reduzieren.

8. Atemunterstützungsgerät nach einem der vorangehenden Ansprüche, wobei der Elektromotor (2) eine Luftdurchlässigkeit zwischen 5 und 50 l/min bei einem Druck von 40 bis 100 mbar aufweist, die durch den Durchmesser (D) und die Anzahl der in den Endflanschen (20a, 20b) des Motorgehäuses (20) ausgebildeten Öffnungen eingestellt wird.

## Claims

1. A respiratory assistance apparatus having an integrated cooling system and comprising an enclosure (100) accommodating a fan (1) that has an electric motor (2) coupled to an impeller (11) and having an inlet (111) and at least two air outlets (121, 122), a gas outlet (121) for the patient and a cooling air outlet (122) of the electric motor (2) which has a longitudinal axis (X'X) and is delimited by a casing (20) having a longitudinal tubular wall (21) and transverse end flanges (20a, 20b), said motor (2) comprising a stator (23) mounted on the tubular wall (21) of the casing (20) and a rotor (22) mounted on a longitudinal motor shaft (25) passing through at least one of the transverse flanges (20a, 20b) of the casing (20) via guide bearings (3) in order to rotate the impeller (11) accommodated in an air-receiving volute (12), in which apparatus the air circulated by the impeller blades (11) is pulsed toward the gas outlet (121) for the patient via an outlet duct (13) coupled to the volute (12), a fraction of this air being drawn from the volute (12) and flowing between the rotor (22) and the stator (23) of the motor (2) through the cooling system that comprises openings (24, 27) formed in each of the transverse flanges (20a, 20b), and an air discharge pipe (105) is coupled both to the tubular wall (21) of the casing (20) and to the cooling air outlet (122) of the enclosure (100), **characterized in that**, in said apparatus, an air flow (F0) coming directly from the inlet (111) of the enclosure (100) is suctioned (arrows F1) into the volute (12) by the rotation of the impeller blades (11), said air flows (arrows F1) coming directly from the space (E1) located above the volute (12), and **in that**, in said system, sealing washers (4a, 4b), secured to the transverse flanges (20a, 20b) so as to cover said flanges, are centered on the motor shaft (25) and are large enough for said washers (4a, 4b) to protect the bearings (3) from the air (F3, F4, F5) flowing through and leaving the motor (2) by extending as close to the motor shaft as possible without generating friction or closing off the openings (24, 27) of the cooling system.

2. Respiratory assistance apparatus according to claim 1, wherein, the motor shaft (25) passing right through the end flange of the motor casing (20) coupled to the volute (12), referred to as the front flange (20a), and penetrating the other end flange, referred to as the rear flange (20b), until it is perpendicular to or below the rear face (2b) of the rear flange (20b), the sealing washer (4a) secured to the front flange (20a) is perforated in its center along a circular cut-out fitted to the motor shaft (25) and the sealing washer (4b) secured to the rear flange (20b) forms a non-perforated cap.

3. Respiratory assistance apparatus according to claim 1, wherein, the motor shaft (25) passing right through the two end flanges (20a, 20b) of the motor casing (20), the sealing washers (4a, 4b) are perforated in their center along a circular cut-out fitted to the motor shaft (25) with substantially zero clearance.

4. Respiratory assistance apparatus according to any of the preceding claims, wherein the washers (4a, 4b) are secured to the end flanges of the motor casing (20) by a securing technique selected from gluing, thermal melting, ultrasonic melting and laser welding.

5. Respiratory assistance apparatus according to any of the preceding claims, wherein the washer or washers (4a, 4b) has or have, after being secured and honed to the motor shaft (25), a depth of cut that is adjusted to the motor shaft (25).

6. Respiratory assistance apparatus according to any of the preceding claims, wherein the openings (24, 27) of the cooling system are evenly and circularly distributed in each of the end flanges (20a, 20b) of the motor casing (20) while being positioned in a longitudinally aligned manner.

7. Respiratory assistance apparatus according to any of the preceding claims, wherein the openings (24, 27) of the cooling system have a diameter (D) suitable for allowing a sufficient cooling air flow rate of the motor (2) without substantially reducing the gas flow rate for the patient.

8. Respiratory assistance apparatus according to any of the preceding claims, wherein the electric motor (2) has an air permeability of between 5 and 50 l/min at a pressure of from 40 to 100 mbar adjusted by the diameter (D) and the number of openings formed in the end flanges (20a, 20b) of the motor casing (20).
